# EUROPEAN PATENT APPLICATION

(11) **EP 4 721 773 A2**
(43) Date of publication of application: **08.04.2026**
(21) Application number: 25222283.1
(22) Date of filing: 24.06.2021
(51) Int. Cl.: A61L 31/14

(54) **COMPOSITE MEDICAL TEXTILE WITH NON-RESORBABLE FIBERS AND BIORESORBABLE HYALURONAN-BASED FIBERS**

(30) Priority: 24.06.2020 US 202063043517 P
(62) Divisional of application: 25157718.5
(71) Applicant: Anika Therapeutics, Inc., Bedford, MA 01730 (US)
(72) Inventor: AGNELLO, Stefano, 35127 Padova (IT); BRACY, Barton W., Orlando, Florida, 32825 (US); BRUNSVOLD, Mark D., Sarasota, Florida, 34242 (US); EK, Steven, Bedford, Massachusetts, 01730 (US); GORALTCHOUK, Alex, Cambridge, Massachusetts, 02141 (US); MANNARINO, Matthew, Bedford, Massachusetts, 01730 (US); MARCHETTO, Elvira, 35127 Padova (IT)
(74) Representative: Peterreins Schley

(57) **Abstract**

A composite medical textile such as a suture includes a plurality of bioresorbable hyaluronan-based fibers and a plurality of non-resorbable fibers. The hyaluronan-based fibers can include at least one of hyaluronic acid, sodium hyaluronate, or the esters of hyaluronic acid such as the benzyl esters. The non-resorbable fibers can be Ultra High Molecular Weight Polyethylene (UHMWPE), and other materials. Methods of making a medical textile are also disclosed.

## Description

### CROSS-REFERENCE TO RELATED APPLICATIONS

This application claims priority to U.S. Provisional Patent Application No. 63/043,517 filed on June 24, 2020, entitled "COMPOSITE SUTURE WITH NON-RESORBABLE FIBERS AND BIORESORBABLE HYALURONAN-BASED FIBERS", the entire disclosure of which incorporated herein by reference.

### FIELD OF THE INVENTION

The present invention relates generally to surgery, and more particularly to medical textiles for medical devices such as sutures, suture tape, sutures with anchors, suture tape with anchors, methods of making sutures, methods of suturing, and generally methods for fixation of tissues.

### BACKGROUND OF THE INVENTION

Medical textiles such as sutures are ubiquitous in the field of surgery. Sutures are typically made from non-resorbable materials such as ultra-high molecular weight polyester (UHMWPE) fibers, as well as polypropylene, nylon, and derivatives thereof. While providing the requisite biocompatibility and strength, these materials must be removed or will remain in place in the patient owing to their non-resorbable nature.

Hyaluronic acid is a naturally occurring non-sulphated glycosaminoglycan consisting of a linear sequence of D-glucuronic acid and N-acetyl-D-glucosamine. It is present in connective tissue, in the synovial fluid of articular joints and in the vitreous humor of the eye. Hyaluronic acid is important in many biological processes such as tissue hydration, cell differentiation, cell behavior and tissue repair. In recent years a hyaluronan polymer-based scaffold has shown surprising properties in the field of tissue engineering. See US 5,939,323 "Hyaluronan Based Biodegradable Scaffolds for Tissue Repair" issued August 17, 1999, and US 6,872,819 "Biomaterials Containing Hyaluronic Acid Derivatives in the Form of Three-Dimensional Structures Free From Cellular Components or Products Thereof for the In Vivo Regeneration of Tissue Cells" issued Mar. 29, 2005. A semisynthetic insoluble polymer has been obtained by the hyaluronic acid esterification with benzyl alcohol. See PCT/EP97/04684 published as WO98/08876 on March 5, 1998 (Fidia Advanced Biopolymers - F.A.B Abano Terme, Italy). The disclosures of these documents are incorporated fully by reference.

Products including hyaluronic acid esterified with benzyl alcohol are sold under the trademark HYAFF11^{®} and are manufactured by Anika Therapeutics S.R.L., Padua Italy. The HYAFF11^{®}composition is biocompatible, completely biodegradable, soluble in dimethylsulfoxide (DMSO), exhibits good stability to hydrolysis, forms contact angle measurements and presents a strong ability to interact with polar molecules. HYAFF11^{®} fibers comprise partially-to-fully esterified hyaluronic acid pendent polymer. This process increases the hydrophobicity of the hyaluronic acid such that it can be produced into non-tissue soluble fiber with a controlled rate of bioresorption. A variety of different chemical compositions of this material can be produced to affect residence time, hydrophilicity, and strength.

### SUMMARY OF THE INVENTION

A composite medical textile includes a plurality of bioresorbable hyaluronan-based fibers and a plurality of non-resorbable fibers. The bioresorbable hyaluronan-based fibers and the non-resorbable fibers can be joined together by at least one selected from the group consisting of braided, knitted, adhered, intermeshed, weaved, interlocked, twisted, and heat set. The medical textile can include from 10 % to 98 % non-resorbable fibers, based on the weight of the non-resorbable fibers to the total weight of the non-resorbable suture fibers and bioresorbable hyaluronan-based polymer fibers.

The hyaluronan-based fibers can include at least one selected from the group consisting of hyaluronic acid, sodium hyaluronate, and esters of hyaluronic acid. The esters of hyaluronic acid can include benzyl esters of hyaluronic acid.

The non-resorbable fibers can include at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.

The medical textile can further include bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxy butyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

The hyaluronan-based bioresorbable polymer fibers can be prepared by at least one selected from the group consisting of ring spinning, air-jet spinning, open-end spinning, mule spinning, wet spinning, dry spinning, electrospinning, pneumatospinning, pultrusion, and extrusion. The extrusion can be at least one selected from the group consisting of solvent exchange extrusion, precipitation extrusion, phase exchange extrusion, and phase change extrusion.

The bioresorbable hyaluronan-based fiber can include at least one selected from the group consisting of mono- and multifilaments. The composite medical textile can have a diameter of from 25 pm to about 5000 pm.

The composite medical textile can include at least one active agent. The active agent can be an antimicrobial agent. The antimicrobial agent can include at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

The active agent can be an analgesic. The analgesic can include at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine.

The active agent can include a vasoconstrictive agent. The vasoconstrictive agent can include at least one selected from the group consisting of epinephrine, alpha-adrenoreceptor antagonists, vasopressin analogues, norepinephrine, phenylephrine, dopamine, dobutamine, serotonin agonists, and triptans.

At least one of the non-resorbable fibers and the bioresorbable hyaluronan-based fibers can be wetted with the active agent. At least one of the non-resorbable fibers and the bioresorbable hyaluronan-based fibers can be coated with the active agent. At least one of the non-resorbable fibers and the bioresorbable hyaluronan-based fibers can have the active agent embedded within the fibers.

The composite medical textile can be circular in cross section. The composite medical textile can be a tape and can have a cross section comprising at least one selected from the group consisting of rounded rectangular, marquise, oblong and oval.

A method of making a medical textile can include the steps of providing a plurality of non-resorbable fibers, and providing a plurality of bioresorbable hyaluronan-based fibers. The non-resorbable fibers and the bioresorbable hyaluronan-based fibers are joined into a composite medical textile. The medical textile can include from 20 % to 80 % bioresorbable hyaluronan-based fibers, based on the total number of non-resorbable fibers and bioresorbable hyaluronan-based fibers.

The composite medical textile can further comprise an active agent, the active agent comprising at least one selected from the group consisting of antimicrobial agents, analgesic agents, and vasoconstrictive agents. The method can include the step of coating the active agent onto at least one selected from the group consisting of the bioresorbable hyaluronan-based fibers and the non-resorbable fibers. The coating step can include at least one selected from the group consisting of a dip, brush, spray, or curtain coating process.

The active agent can be impregnated into at least one selected from the group consisting of the bioresorbable hyaluronan-based fibers and the non-resorbable fibers by passing the extruded fiber through a solution containing the active agent. The active agent can be impregnated into at least one selected from the group consisting of the bioresorbable hyaluronan-based fibers and the non-resorbable fibers by co-extrusion, wherein a fiber precursor and the active agent are combined into a homogeneous mixture and co-extruded into a fiber. At least one of the bioresorbable hyaluronan-based fibers and the non-resorbable fibers can be wetted with the active agent.

A method for repairing a portion of a mammalian body can include the steps of providing a composite medical textile which comprises a plurality of non-resorbable fibers and a plurality of bioresorbable hyaluronan-based fibers, and connecting the textile between two tissue portions of the mammalian body. The tissue portions can include at least one selected from the group consisting of bony tissue and soft tissue. The medical textile can be a suture, and the method can further include the step of manipulating the suture in a suturing process to suture the tissue portions of the mammalian body. The method can also include the step of seeding the medical textile with stem cells.

A medical device according to the invention can include a plurality of bioresorbable hyaluronan-based fibers and a plurality of non-resorbable fibers. The medical device can be an orthopedic attachment system which comprises at least one flexible connector comprising bioresorbable hyaluronan-based fibers joined with a plurality of non-resorbable fibers, and at least one orthopedic attachment device. The medical device can be tubes, membranes, non-woven fabrics, gauzes, sponges, and/or sutures. The medical device can be a tissue scaffold.

### BRIEF DESCRIPTION OF THE DRAWINGS

There are shown in the drawings embodiments that are presently preferred it being understood that the invention is not limited to the arrangements and instrumentalities shown, wherein:
Figure 1 A is a perspective schematic diagram of a suture anchor with a composite suture according to the invention.
Figure 1 B is a perspective schematic diagram of the suture anchor with a composite suture of indefinite length according to the invention.
Figures 2 A-C are schematic diagrams of: FIG. 2A - untwisted filament yarn; FIG. 2B - twisted filament yarn; and FIG. 2C - high bulk filament yarn.
Figures 3 A-E are schematic diagrams of a medical textile according to the invention showing: FIG. 3A - nonwoven; FIG. 3B - weave; FIG. 3C - braid; FIG. 3D - weft-knit; and FIG. 3E - warp knit.
Figure 4 is a perspective view of a soft tissue anchor assembly according to the invention;
FIG. 4A is an expanded view of area 4A in FIG. 4; and FIG. 4B is an expanded view of area 4B in FIG. 4.
Figure 5 is a perspective view of a rigid bone anchor assembly according to the invention, in a first mode of operation; and FIG. 5A is an expanded view of area 5A in FIG. 5.
Figure 6 is a perspective view of the rigid bone anchor assembly of FIG. 5 in a second mode of operation; and FIG. 6A is an expanded view of area 6A in FIG. 6.
Figure 7 is a side elevation of a joint repair assembly according to the invention; and FIG. 7A is an enlarged view of area 7A in FIG. 7.
Figure 8 is a side elevation of a tissue anchor according to the invention; FIG. 8A is an enlarged view of area 8A in FIG. 8; and FIG. 8B is an enlarged view of area 8B in FIG. 8.
Figure 9 is a side elevation of the tissue anchor of FIG. 8, in a deployed mode of operation.
Figure 10 is a side elevation of a braided medical textile according to the invention; and FIG. 10A is a cross-section taken along line 10A-10A in FIG. 10.
Figure 11 is an enlarged side elevation view of area FIG. 11 in FIG. 10.
Figure 12 is a schematic cross-section of a medical textile according to the invention in an initial mode of operation.
Figure 13 is a schematic cross-section of the medical textile of FIG. 12 showing degradation of bioresorbable fibers and cell growth.
Figure 14 is a schematic cross-section of a medical textile with a mixed array of hyaluronan-based bioresorbable fibers and non-resorbable fibers.
Figure 15 is a schematic cross-section of the medical textile with an array of hyaluronan-based bioresorbable fibers surrounding non-resorbable fibers.
FIG 16 is a schematic cross-section of a medical textile with an array of hyaluronan-based bioresorbable fibers surrounded by non-resorbable fibers.
FIG 17 is a schematic cross-section of a medical textile in which hyaluronan based bioresorbable fibers of a larger diameter are mixed with resorbable fibers of a smaller diameter.
Figure 18 is a schematic cross-section of a medical textile in which hyaluronan based bioresorbable fibers and non-resorbable fibers are mixed with another fiber which can be bioresorbable or non-resorbable.
Figure 19 is a schematic cross-section of the medical textile surrounded by a coating of an active material.
FIG 20 is a schematic cross-section of a medical textile in which one or both of the hyaluronan based bioresorbable fibers or non-resorbable fibers are coated with an active material.
Figure 21 is a plot of knot pull strength (N) versus time (days) for a suture according to the invention and two prior art sutures.

### DETAILED DESCRIPTION OF THE INVENTION

A composite medical textile such as a suture according to the invention includes a plurality of bioresorbable hyaluronan-based fibers interlocked with a plurality of non-resorbable fibers. A plurality of different kinds of hyaluronan-based fibers can be used with a plurality of different kinds of bioresorbable fibers. The term "hyaluronan-based" as used herein encompasses fiber materials that can include at least one selected from the group consisting of hyaluronic acid, sodium hyaluronate, and benzyl esters of hyaluronic acid, such as but not limited to the benzyl ester derivative of hyaluronic acid sold as HYAFF^{®}. Other esters of hyaluronic acid with aliphatic, araliphatic, cycloaliphatic or heterocyclic alcohols, in which are esterified all (so-called "total esters") or only a part (so-called "partial esters") of the carboxylic groups of the hyaluronic acid are also possible. Crosslinked hyaluronic acid-based fibers that are cross-linked with such compounds as bis(ethylcarbodiimide)(BCDI), formaldehyde and other aldehydes, (1-Ethyl-3-(3-dimethylaminopropyl)carbodiimide)(EDC), dicyclohexylcarbodiimide)(DCC) or other carbodiimide crosslinking agents, and click chemistry, can also be used. All of the above are hyaluronan-based materials as used herein. Combinations of hyaluronan-based materials are also possible.

The hyaluronan-based material can be the sodium salt of hyaluronic acid, sodium hyaluronate, but the acid will usually also be present to some degree. Sometimes up to 3% or more of the acid or conjugate is present. The acid will also be present to some degree with HYAFF^{®} materials. The degree of substitution of HYAFF^{®} materials varies between approximately 50 and approximately 100%. The degree of substitution of the hyaluronic acid can be 50, 55, 60, 65, 70, 75, 80, 85, 90, 95, 96, 97, 98, 99 and 100 %, and can be within a range of any high value and low value selected from these values. The remaining functional groups are either all acid, all base, or a mix of acid and base. Blends of fibers having different degrees of substitution are also possible, and crosslinked hyaluronic acid. The total benzyl esterified form, named HYAFF 11 ^{®}p 100, can be used. HYAFF11 ^{®} p80 and HYAFF11 ^{®} p75 is partially esterified and can also be used (Anika Therapeutics S.R.L., Padua Italy).

The individual fiber dimensions can vary. The deniers (D, g/9000 m) of the non-resorbable and hyaluronan-based bioresorbable fibers can vary from 250 to 100,000 D. The denier range can be from 500 to 15,000 D. Some bioresorbable fibers are from 540 to 720 D. The deniers of the non-resorbable fibers and the hyaluronan-based bioresorbable fibers can be, independently, 250, 300, 400, 500, 750, 1000, 2000, 3000, 4000, 5000, 6000, 7000, 8000, 9000, 10000, 11000, 12000, 13000, 14000, 15000, 16000, 17000, 18000, 19000, 20000, 30000, 40000, 50000, 60000, 70000, 80000, 90000, and 100000 D, and can be within a range of any high value and low value selected from these values. Individual fibers of specific deniers can be combined in a composite suture arrangement of multi-filament, multi-denier bundles.

The diameter of the individual non-resorbable and hyaluronan-based bioresorbable fibers can be from 15-250 pm. The diameter can be from 50-60 pm. The diameter of the non-resorbable and hyaluronan-based bioresorbable fibers can be, independently, 15, 20, 25, 30, 40, 50, 60, 70, 80, 90, 100, 110, 120, 130, 140, 150, 160, 170, 180, 190, 200, 210, 220, 230, 240, and 250 pm, and can be within a range of any high value and low value selected from these values. Individual fibers of specific diameters can be combined in a composite suture arrangement of multi-diameter fibers.

The weight proportion of non-resorbable fibers to the total weight of the non-resorbable fibers and the bioresorbable hyaluronan-based fibers can be 10%-98%. The weight proportion of non-resorbable fibers to the total weight of the non-resorbable fibers and the bioresorbable hyaluronan-based fibers can be from 30%-80%. The weight proportion of non-resorbable fibers to the total weight of the non-resorbable fibers and the bioresorbable hyaluronan-based fibers can be 10, 15, 20, 25, 30, 35, 40, 45, 50, 55, 60, 65, 70, 75, 80, 85, 90, 95, 96, 97, and 98%, and can be within a range of any high value and low value selected from these values.

A composite suture made from the medical textile of the invention comprising both the non-resorbable fibers and the bioresorbable hyaluronan-based fibers can have different dimensions. Typical suture is commonly from United States Pharmacopeia (USP) sizes USP 3-0 to USP 5. The composite suture can have a size range of from 100 to 5000 pm, more specifically 25 to 750 pm. A common size is from 50 to 65 pm. Some special use suture can be up to 5000 pm. The composite suture can have a diameter of 25, 50, 75, 100, 200, 300, 400, 500, 600, 700, 800, 900, 1000, 1250, 1500, 1750, 2000, 2250, 2500, 2750, 3000, 3250, 3500, 3750, 4000, 4250, 4500, 4750, and 5000 pm, and can be within a range of any high value and low value selected from these values. It is common that suture dimensions will vary slightly due to small variations in fiber dimensions and assembly conditions, and accordingly the above sizes can be considered as averages.

The number of fibers in the suture composite can vary. The suture composite can have 25 to 10,000 total individual fibers, including both the bioresorbable fiber strands and the hyaluronan-based bioresorbable fiber strands. The number of total fiber strands can be from 150 to 6000 individual fiber strands. The total number of fiber strands can be 25, 50, 75, 100, 200, 300, 400, 500, 600, 700, 800, 900, 1000, 2000, 3000, 4000, 5000, 6000, 7000, 8000, 9000, and 10000 strands, and can be within a range of any high value and low value selected from these values.

The number proportion of non-resorbable suture fibers to bioresorbable hyaluronan-based polymer fibers in the composite suture can vary. The composite suture can include from 20 % to 80 % bioresorbable hyaluronan-based polymer fibers, based on the total number of non-resorbable suture fibers and bioresorbable hyaluronan-based polymer fibers. The composite suture can include 20 %, 25 %, 30 %, 35 %, 40 %, 45 %, 50 %, 55 %, 60 %, 65 %, 70 %, 75 %, or 80 % bioresorbable hyaluronan-based polymer fibers, based on the total number of non-resorbable suture fibers and bioresorbable hyaluronan-based polymer fibers. The number proportion of bioresorbable hyaluronan-based polymer fibers, based on the total number of non-resorbable suture fibers and bioresorbable hyaluronan-based polymer fibers, can be within a range of any high value and low value selected from these values.

The medical textile can have a straight tensile strength of from 15 to 800 N. The medical textile can have a straight tensile strength of 15, 20, 25, 30, 35, 40, 60, 80, 100, 120, 140, 160, 180, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, and 800 N, and can be within a range of any high value and low value selected from these values.

The medical textile when formed as a suture can have a knot pull strength of from 40 to 300 N. The knot pull strength can be 40, 50, 60, 70, 80, 90, 100, 110, 120, 130, 140, 150, 160, 170, 180, 190, 200, 210, 220, 230, 240, 250, 260, 270, 280, 290, and 300 N, and can be within a range of any high value and low value selected from these values.

The bioresorbable fibers can resorb within about 2 - 8 months. The time for resorption of the bioresorbable fibers can be 2, 3, 4, 5, 6, 7, or 8 months, or within a range of any high value and low value selected from these values.

The non-resorbable fibers can be formed from different materials. The non-resorbable fibers can comprise ultra-high molecular weight polyethylene (UHMWPE). The material of the non-resorbable suture fiber can also include polypropylene, polyethylene terephthalate, polyethylene, polytetrafluoroethylene (Teflon), Dacron, steel, polybutester, polyamide, polyester, polyurethane, nylon and its derivatives, silk and cotton. Combinations of non-resorbable materials are also possible.

The hyaluronan-based bioresorbable polymer fibers can be prepared by different processes. The hyaluronan-based bioresorbable fibers can prepared by at least one selected from the group consisting of ring spinning, air-jet spinning, open-end spinning, mule spinning, wet spinning, dry spinning, electrospinning, pneumatospinning, pultrusion, and extrusion. The extrusion process can be at least one selected from the group consisting of solvent exchange extrusion, precipitation extrusion, phase exchange extrusion, and phase change extrusion. Other processes are possible. The hyaluronan-based bioresorbable fibers can be mono filaments, and can be multifilaments.

Additional bioresorbable fibers can be used with the hyaluronan-based bioresorbable fibers. Such additional bioresorbable fibers include bioresorbable polymer fiber comprising at least one of collagen, polylactic acid (PLA), polyglycolic acid (PGA), polylactic-co-glycolic acid (PLGA), polycaprolactone (PCL), polydioxanone (PDO), polyhydroxy butyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and/or butyric acid.

The composite suture of the invention can also include at least one active agent. Many different kinds of active agents are possible. The active agent can be an antimicrobial agent. The antimicrobial agent can include at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.

The active agent can be an analgesic. The analgesic can be at least one selected from the group consisting of Lidocaine, Bupivacaine, amylocaine, articaine, aspirin, benzocaine, benzonatate, butacaine, butanilicaine, chloroprocaine, clopidogrel, cinchocaine, cyclomethycaine, eucaine, ibuprofen, naproxen, paclitaxel, warfarin, heparin, tetracaine, dexamethasone, and ropivocaine.

The active agent can be a vasoconstrictive agent. The vasoconstrictive agent can include at least one selected from the group consisting of epinephrine, alpha-adrenoreceptor antagonists, vasopressin analogues, norepinephrine, phenylephrine, dopamine, dobutamine, serotonin agonists, and triptans.

The active agent can be included in the composite suture by different processes. The active agent can be applied to at least one of the non-resorbable fibers and the bioresorbable hyaluronan-based fibers by wetting with the active agent. The active agent can be applied to at least one of the non-resorbable fibers and the bioresorbable hyaluronan-based fibers by coating with the active agent. The active agent can be applied to at least one of the non-resorbable fibers and the bioresorbable hyaluronan-based fibers by embedding the active agent into the fiber during the extrusion of the fiber. The active agent can be chemically or ionically cross-linked or grafted to the surface of at least one of the non-resorbable fibers and the bioresorbable hyaluronan-based fibers.

The coating step can be performed by any suitable process. The coating step can be at least one selected from the group consisting of a dip, brush, spray, or curtain coating process.

The active agent can be impregnated into at least one selected from the group consisting of the bioresorbable hyaluronan-based fibers and the non-resorbable fibers by passing the extruded fiber through a solution containing the active agent. The impregnating step can be by co-extrusion, wherein a fiber precursor and the active agent are combined into a homogeneous mixture and co-extruded into a fiber.

The composite suture can have different cross-sectional shapes. The composite suture can be circular in cross section. The composite suture can be a tape and have a cross section comprising at least one selected from the group consisting of rounded rectangular, marquise, oblong and oval. A method of making a suture fiber can include the step of providing a plurality of non-resorbable fibers and a plurality of bioresorbable hyaluronan-based fibers. The non-resorbable fibers and the bioresorbable hyaluronan-based fibers are interlocked into a composite suture. The joining of the bioresorbable hyaluronan-based fibers and the non-resorbable fibers can be by any suitable process. Examples of suitable processes include twisting, weaving, braiding, knitting, adhering and heat treating.

The medical textile of the invention can take many different textile forms. The bioresorbable hyaluronan-based fibers and non-resorbable fibers can be knitted, twisted, braided, non-woven or other forms of textile construction for combining diverse fibers. Braided suture can for example contain bioresorbable hyaluronan-based fibers, non-resorbable fibers, and one or more additional fibers, which can be bioresorbable fibers and/or non-resorbable fibers. Braided suture can contain a core filament such as bioresorbable hyaluronan-based fibers and an outer braided sheath of one or more polymers. Braided suture can be coated with a bioresorbable hyaluronan-based film. Braided or woven suture can contain bioresorbable hyaluronan-based fibers and at least one other fiber, which can be another bioresorbable hyaluronan-based fiber and/or a non-resorbable fiber. Other medical textile constructions of bioresorbable hyaluronan-based fibers and non-resorbable fibers are possible.

The medical textiles of the invention can be used in conjunction with multiple types of fixation devices, known in the field of orthopedics for repairing damaged soft tissue. These fixation devices can be bone screws, bone screws with washers, suture anchors, suture buttons and the like; used to create, repair or augment tissue connection sites relevant to orthopedic procedures. These connection sites can be comprised of bone-to-bone tissue connections, as typically seen in ligament injuries; tissue-to-bone connections, as typically seen in tendon or ligament injuries; and tissue-to-tissue connections, as typically seen within tendon, ligament, or muscle injuries. The medical textiles used in conjunction with these fixation devices may take the form of sutures or suture tapes of different constructions and sizes.

The medical textiles of the invention can be fashioned so that they are packaged and provided to the surgeon user directly coupled to these fixation devices, or they can be fashioned using splicing or knotting techniques within the textile so that they could be packaged and provided to the surgeon user for use with separately packaged fixation devices.

The medical textile can also be used to make scaffolds. Scaffolds made with the medical textiles of the invention will provide requisite initial and long-term strength by the presence of both hyaluronan-based bioresorbable fibers and non-resorbable fibers and will allow for cell growth into and around the scaffold. Scaffold devices include tubes, membranes, non-woven fabrics, gauzes, and sponges.

Braided surgical sutures have been shown to cause more trauma (abrasion, tearing, cutting, etc.) to soft tissue than monofilament suture. Kowalsky MS, Dellenbaugh SG, Erlichman DB, et al., "Evaluation of suture abrasion against rotator cuff tendon and proximal humerus bone" Arthroscopy. 2008;24:329-334. It is also known in the medical field that a lubricant coating on braided sutures reduces the friction as well as the tissue drag which can lead to tearing and trauma on soft tissue; however many lubricant coatings are either short-lived in vivo or are composed of potentially toxic materials. The use of conventional resorbable polymers (e.g., PLA, PGA, PCL, PDO, PHBV, etc.) in a composite braided suture structure will degrade by hydrolysis into smaller molecules and/or their respective monomers. The use of esterified hyaluronic acid such as HYAFF11^{®} (Anika Therapeutics S.R.L., Padua Italy) in a composite braided structure will degrade into benzyl alcohol and high molecular weight hyaluronic acid (HA) overtime in vivo. HA is a naturally occurring polysaccharide with unique viscoelastic and rheological properties; it serves as a lubricating agent on articular tissues and prevents mechanical damage. Its viscoelasticity has been shown to be responsible for protecting, lubricating, and stabilizing cells and tissue layers during joint movement. Goa, Karen L., and Paul Benfield. "Hyaluronic acid." Drugs 47.3 (1994): 536-566. Furthermore, high molecular weight HA has been shown to provide an anti-inflammatory response in addition to improved lubrication properties in tendon lesions. Necas, J. B. L. B. P., et al. "Hyaluronic acid (hyaluronan): a review." Veterinarni medicina 53.8 (2008): 397-411.

In the embodiment of a composite braided, woven, or twisted medical textile such as suture or suture tape comprising of esterified HA (such as HYAFF11^{®}p100 Anika Therapeutics S.R.L., Padua Italy) fibers and non-absorbable polymer (e.g., UHMWPE) fibers, the Hyaff material will provide a slow release of a self-lubrication agent (high MW HA) to the braided suture structure. The release of HA overtime will provide a self-replenishing supply of lubricant that will reduce abrasion and soft tissue trauma as well as reduce the inflammatory response, which could improve the healing response of the repaired soft tissue.

Hyaluronic acid (HA) is a naturally occurring polysaccharide that is an important component of extra-cellular matrix (ECM); it interacts with binding proteins, proteoglycan, growth factors and other active molecules and contributes to the regulation of water balance. HA is present in all adult joint tissues, including synovial fluid and its rheological properties in solution make it an ideal a lubricant for protecting articular cartilage. Furthermore, HA acts as scavenger molecule for free radicals, inhibits leukocyte and macrophage migration, and helps regulate fibroblast proliferation. Besides all these properties, HA is recognized by some cell receptors such as CD44, regulating the adhesion, differentiation, angiogenesis and modulating the inflammation. For these and other biological properties, HA represents an optimal candidate as a biomaterial to produce scaffolds; however, its water solubility, rapid resorption, and short residence time in the tissue, limit its possible application. Campoccia D. et al., Biomaterials, 19 (1998) 2101-2127. Different HA chemical modification techniques have been used to improve the physical and mechanical properties. One of the most promising materials for tissue engineering and regenerative medicine is the benzyl ester derivative of hyaluronan (HYAFF11^{®}). One of the main advantages of HYAFF 11^{®} based scaffold is the degree of cell adhesiveness even without any coating or treatment with different molecules, generally required by other biomaterials such as polyglycolide and polylactide. The total benzyl esterified form of HYAFF11^{®}p100 is sufficiently stable in an aqueous environment, maintaining its structural integrity, so it is easy to handle and does not contract as some collagen-based materials. Campoccia D. et al., Biomaterials, 19 (1998) 2101-2127. HYAFF11^{®}p100 can be processed to obtain several types of devices such as tubes, membranes, non-woven fabrics, gauzes, and sponges. All these scaffolds are highly biocompatible. In the human body they do not elicit any adverse reactions and are resorbed by the host tissues. Vindigni V. et al., Int. J. Mol. Sci., 10, (2009) 2972-2985.

HYAFF11^{®} scaffolds have been shown to be suitable supports for the attachment, growth, and proliferation of mammalian cells. Human preadipocytes can be successfully and reproducibly inoculated and cultured on HYAFF 11^{®}-based three-dimensional scaffolds where there was clear evidence that adipocyte precursor cells placed on this material were able to undergo full maturation into adipocytes. Halbleib, M. et al., Biomaterials, 24, (2003) 3125-3132. Additionally, human hepatocytes, dermal fibroblasts and keratinocytes, chondrocytes, Schwann cells, bone marrow derived mesenchymal stem cells and adipose tissue derived mesenchymal stem cells have been successfully cultured in HYAFF11 ^{®} meshes. Vindigni V. et al., Int. J. Mol. Sci., 10, (2009) 2972-2985. Furthermore, HYAFF11^{®} scaffolds support the adhesion, migration and proliferation of rMSCs, as well as the synthesis and delivery of extracellular matrix components under static culture conditions without any chemical induction. The high retention rate and viability of the seeded cells as well as their fine modality of interaction with the substrate suggest that such scaffolds could be potentially useful when wide tissue defects are to be repaired as in the case of cartilage repair, wound healing and large vessel replacement. Pasquinelli G. et al., J. Anat., 213 (2008) 520-530.

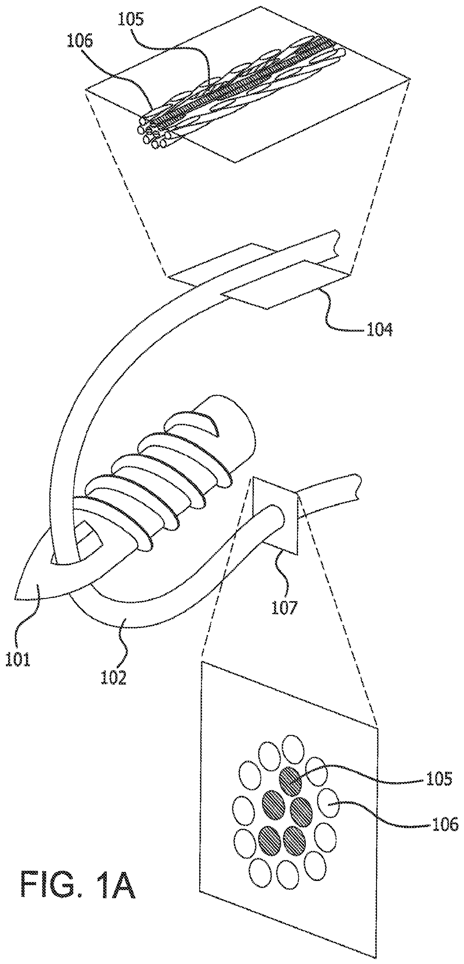

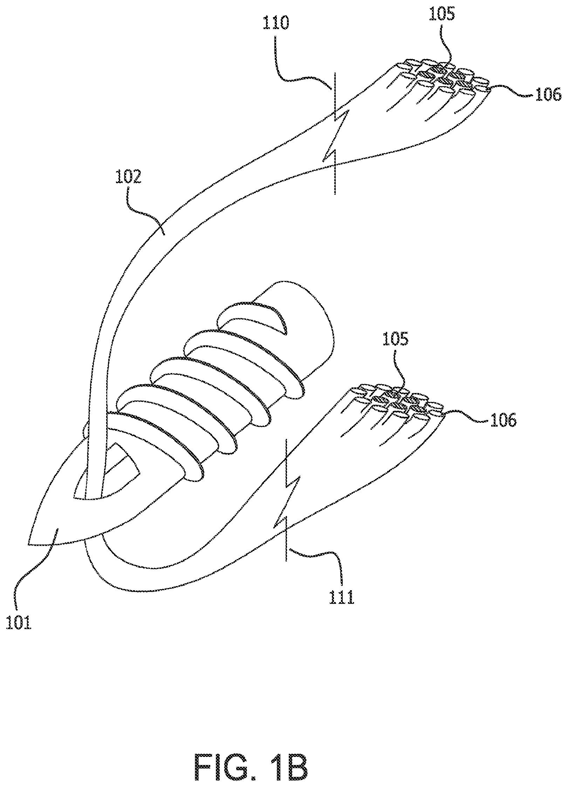

Figure 1A is a perspective schematic diagram of suture anchor 101 with a composite suture 102 according to the invention. The composite suture 102 according to the invention has, as shown in longitudinal cross section taken at 104 and lateral cross section taken at 107, a hyaluronan-based fiber bundle 105 and a non-resorbable fiber bundle 106. The suture can be off indefinite length as indicated by break-away lines 110 and 111 in FIG. 1B. The anchor 101 can be biostable or bioresorbable.

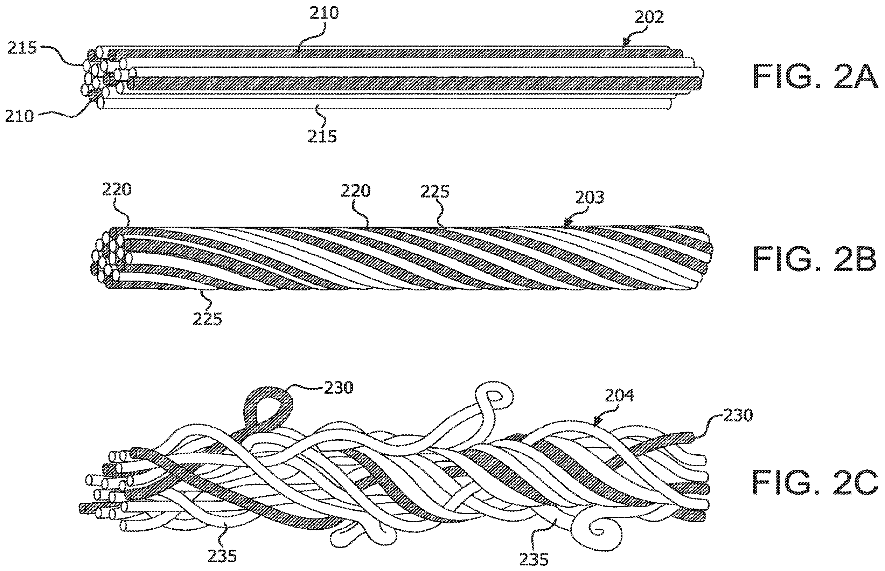

Figure 2A is a schematic diagram of untwisted filament yarn 202 having hyaluronan-based bioresorbable fibers 210 and non-resorbable fibers 215. Figure 2B is an illustration of twisted filament yarn 203 having hyaluronan-based bioresorbable fibers 220 and non-resorbable fibers 225. Figure 2C is a schematic picture of a high bulk filament yarn 204 having hyaluronan-based bioresorbable fibers 230 and non-resorbable fibers 235. Other constructions are possible.

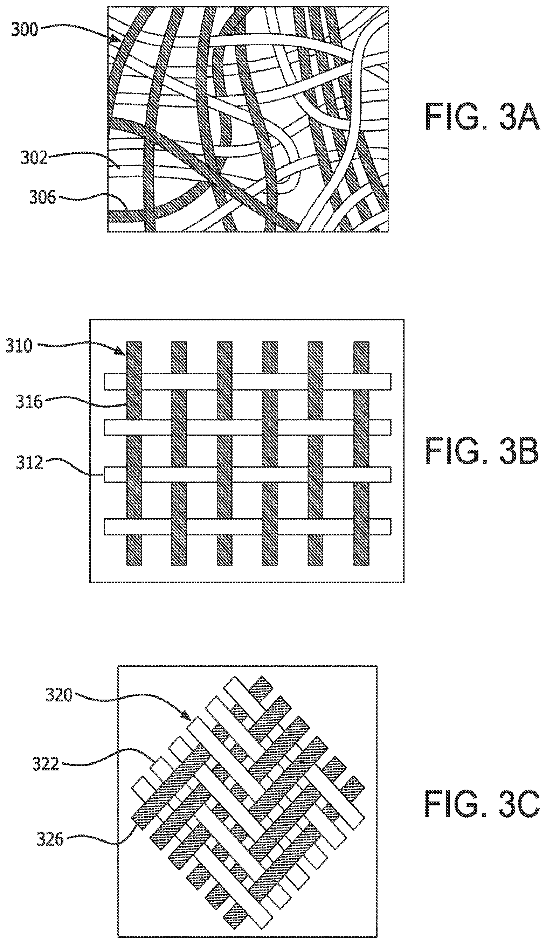

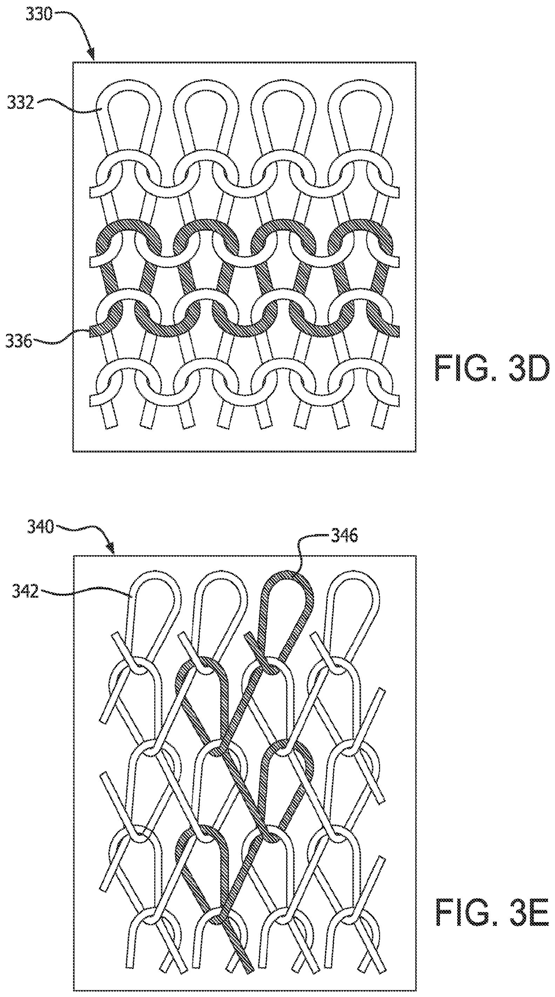

Figure 3A is a schematic diagram illustrating a nonwoven medical textile 300 according to the invention with bioresorbable hyaluronan-based fibers 306 and non-resorbable fibers 302. Figure 3B is a schematic diagram of a woven medical textile 310 with hyaluronan-based bioresorbable fibers 316 and non-resorbable fibers 312. Figure 3C is a schematic diagram of a braided construction 320 with hyaluronan-based bioresorbable fibers 326 and non-resorbable fibers 322. Figure 3D is a schematic representation of a weft-knit medical textile 330 having hyaluronan-based bioresorbable fibers 336 and non-resorbable fibers 332. Figure 3E is a schematic diagram of a warp knit medical textile 340 having hyaluronan-based bioresorbable fibers 346 and non-resorbable fibers 342.

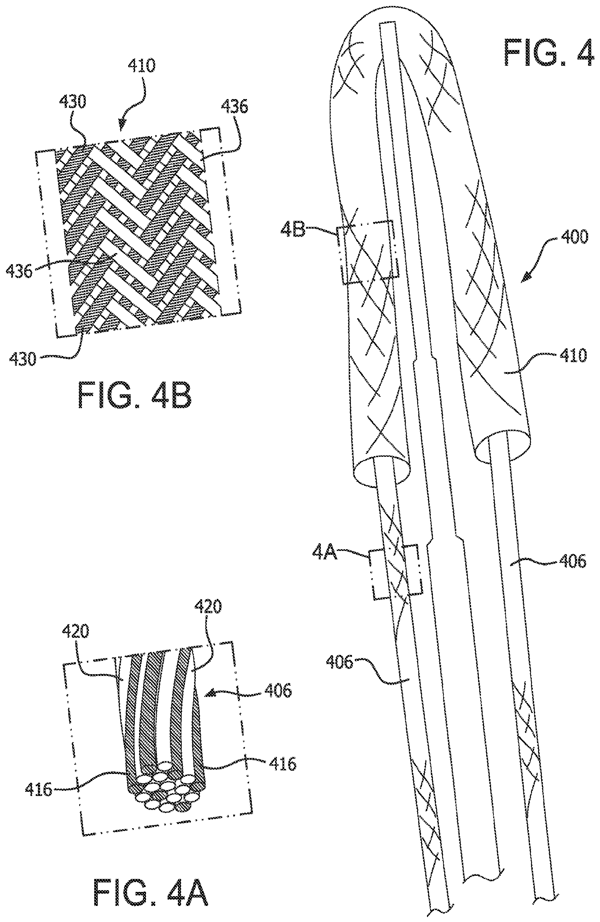

Figure 4 is a perspective view of a soft anchor assembly 400 according to the invention. The soft anchor assembly 400 has a suture 406 which passes through a flexible tubular anchor 410. As shown in FIG. 4A, the suture 406 can be comprised of a plurality of hyaluronan-based bioresorbable fibers 416 and non-resorbable fibers 420. As shown in FIG. 4B, the tubular anchor 410 can be comprised of a braided construction of hyaluronan-based bioresorbable fibers 430 and non-resorbable fibers 436. The construct can be varied, for example by making one of the tubular anchor 410 and the suture 406 completely from non-resorbable fibers or from bioresorbable fibers such as the hyaluronan-based bioresorbable fibers.

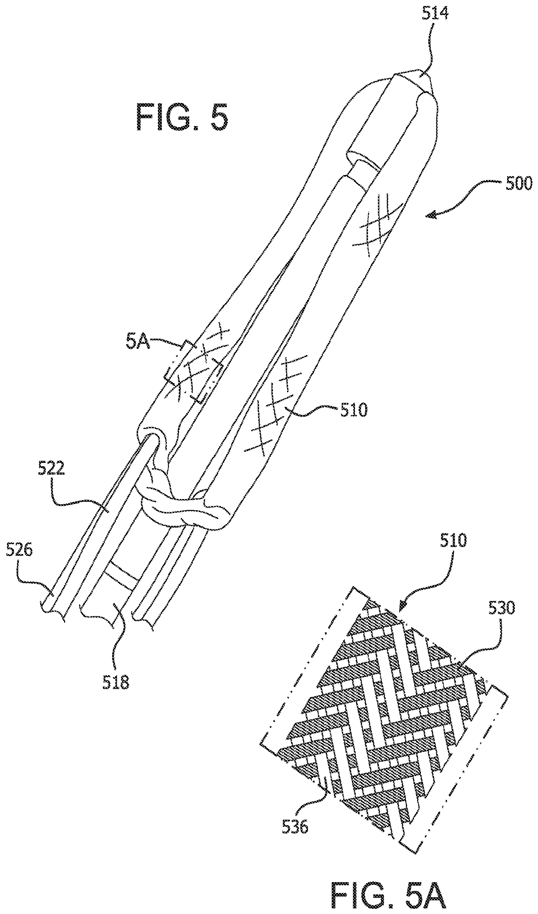

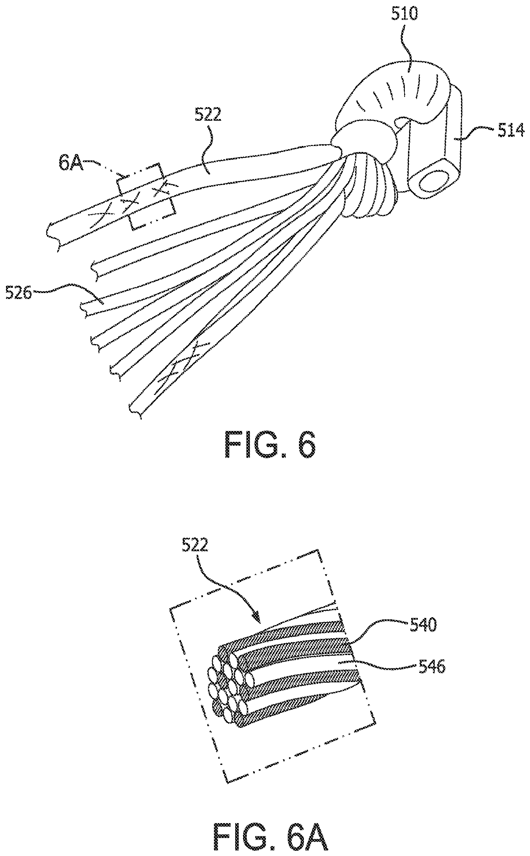

Figure 5 is a perspective view of a rigid bone anchor assembly 500 according to the invention, in a first mode of operation. The rigid bone anchor assembly 500 includes a flexible tubular locking material 510 communicating with an aperture in a rigid anchor 514. Anchor securing sutures 522 and tissue fixation sutures 526 can also be provided. A deployment tool 518 can be used to deploy the anchor assembly 500. As shown in FIG. 5A, the flexible tubular anchor 510 can be comprised of a braided medical textile of hyaluronan-based bioresorbable fibers 530 and non-resorbable fibers 536. Figure 6 is a perspective view the rigid bone anchor assembly 500, in a deployed mode of operation. As shown in FIG. 6A, the anchor securing sutures 522 can be comprised of a twisted assembly of hyaluronan-based bioresorbable fibers 540 and non-resorbable fibers 546. An assembly of hyaluronan-based bioresorbable fibers and non-resorbable fibers can also be used for the tissue fixation sutures 526.

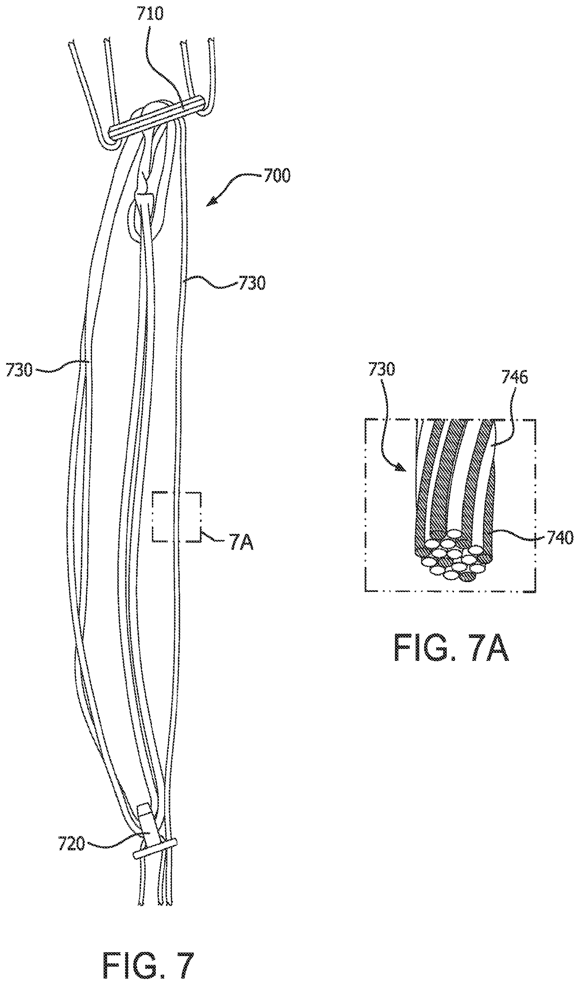

Figure 7 is a side elevation of a joint repair assembly 700 according to the invention having surgical button 710, fixation device 720, and connecting suture 730. The suture 730 can include an assembly of hyaluronan-based bioresorbable fibers 740 and non-resorbable fibers 746, as shown in FIG. 7A.

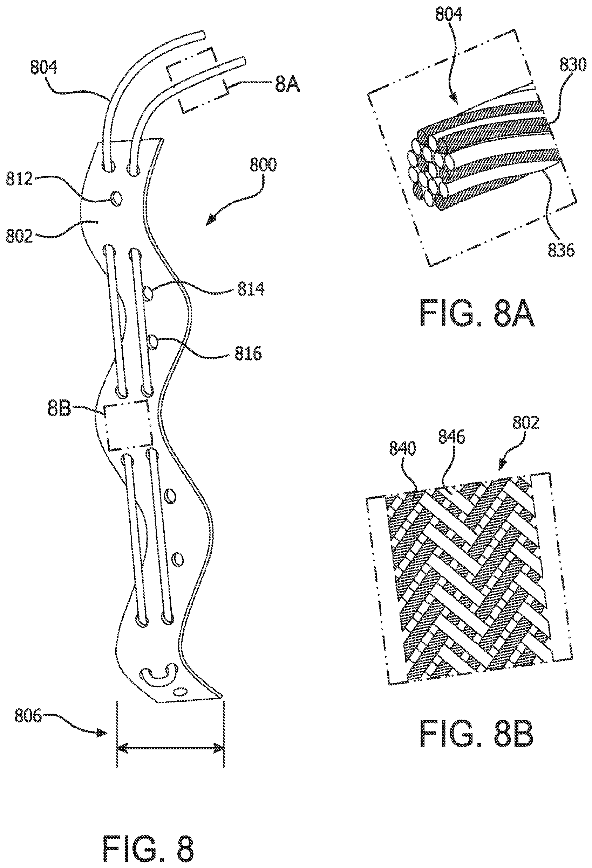

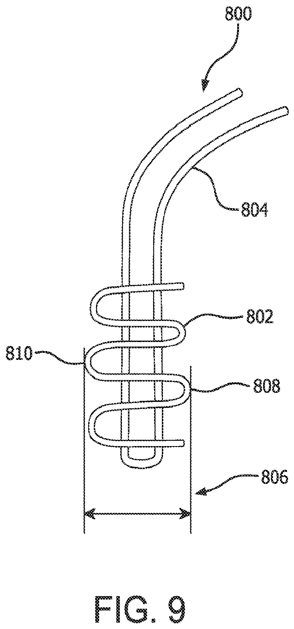

Figure 8 is a side elevation of a tissue anchor 800 the general features of which are shown and described in US 10,918,372 "Suture Anchor" issued Feb. 16, 2021. The tissue anchor 800 includes a webbing portion 802 and a deployment suture 804. In some embodiments the webbing portion 802 will include a plurality of apertures 812, 814, 816 within which an insertion tool may be temporarily disposed. As shown in FIG. 8A, the deployment suture 804 can be comprised of a plurality of hyaluronan-based bioresorbable fibers 830 and non-resorbable fibers 836. The webbing portion 802 can also be comprised of hyaluronan-based bioresorbable fibers 840 and non-resorbable fibers 846 as shown in FIG. 8B. The dimension 806 can expand as the webbing portion 802 transitions from the un-constricted configuration to the constricted configuration shown in FIG. 9. Surface regions 808 and 810 will be urged radially outwardly to engage the surrounding matrix of bone, soft tissue or other media.

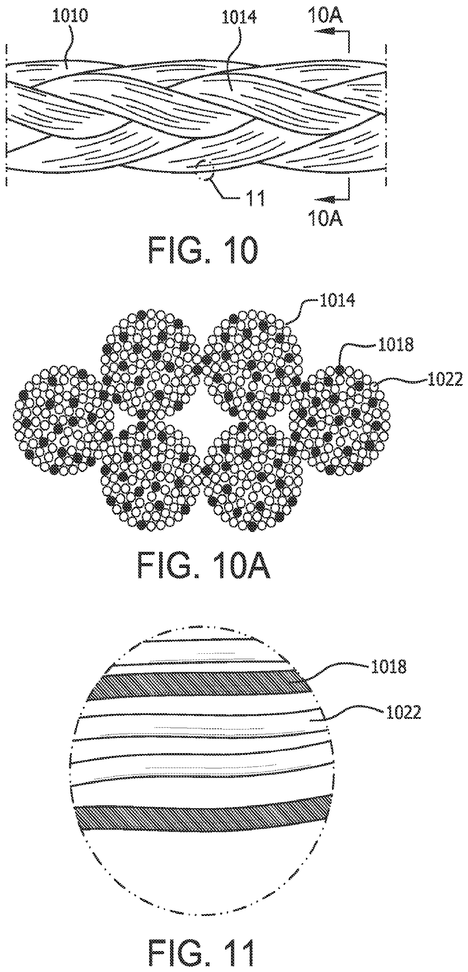

Figure 10 is a side elevation of a braided medical textile 1010 which is comprised of braided strands 1014. As shown in FIG. 10A, each of the strands 1014 is comprised of a plurality of hyaluronan-based bioresorbable fibers 1018 and non-resorbable fibers 1022. Figure 11 is an enlarged view of area FIG. 11 in FIG. 10. The hyaluronan-based bioresorbable fibers 1018 closely adjoin the non-resorbable fibers 1022 and significant inter-fiber friction is possible. Such inter-fiber friction is reduced by the hyaluronan-based fibers 1018, which as noted above are self-lubricating.

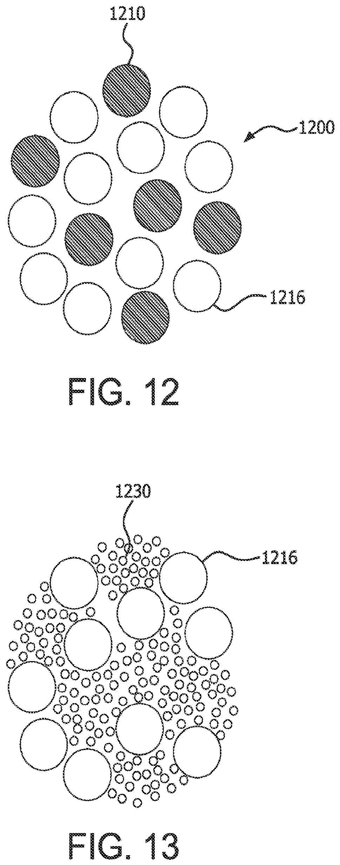

Figure 12 is a schematic cross-section of a medical textile 1200 according to the invention. The medical textile 1200 includes a plurality of hyaluronan-based bioresorbable fibers 1210 and non-resorbable fibers 1216. As shown in FIG. 13, in time the hyaluronan-based bioresorbable fibers 1210 will fully resorb and cells 1230 from the patient will propagate in the location of the resorbed hyaluronan-based bioresorbable fibers 1210. Also, stem cells can be positioned to propagate as the hyaluronan-based fibers resorb.

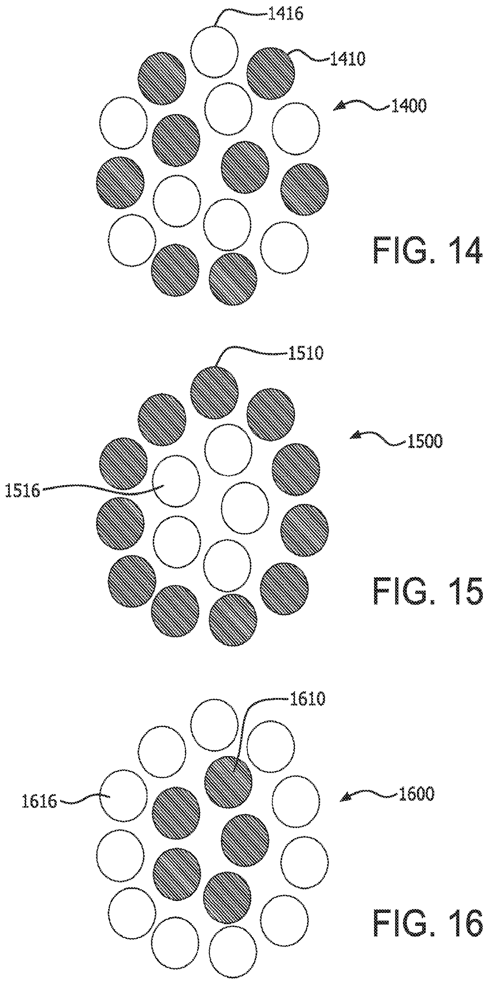

Many different configurations of hyaluronan-based bioresorbable fibers and non-resorbable fibers are possible in medical textiles according to the invention. Figure 14 is a schematic cross-section of a medical textile 1400 with a mixed plurality of hyaluronan-based bioresorbable fibers 1410 and non-resorbable fibers 1416.

Figure 15 is a schematic cross-section of the medical textile 1500 with a plurality of hyaluronan-based bioresorbable fibers 1510 surrounding non-resorbable fibers 1516.

FIG 16 is a schematic cross-section of a medical textile 1600 with an array of hyaluronan-based bioresorbable fibers 1610 surrounded by non-resorbable fibers 1616.

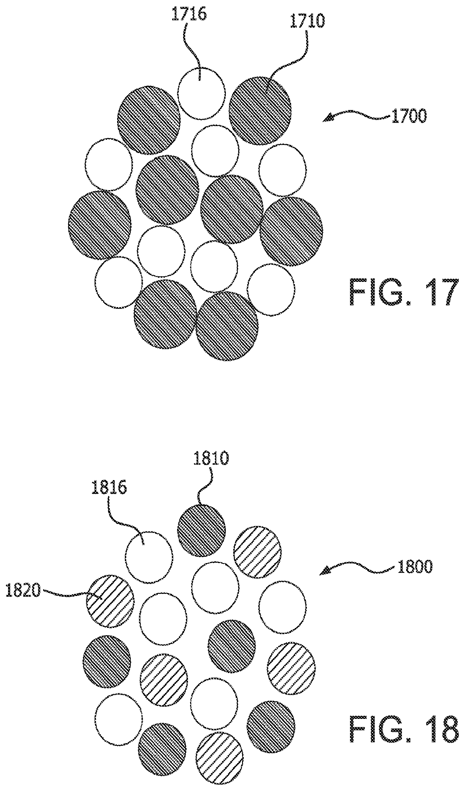

FIG 17 is a schematic cross-section of a medical textile 1700 with a plurality of hyaluronan-based bioresorbable fibers 1710 with non-resorbable fibers 1716. The hyaluronan-based bioresorbable fibers 1710 have a larger diameter than the diameter of the bioresorbable fibers 1716. It is also possible that the hyaluronan-based bioresorbable fibers could have a smaller diameter than the diameter of the non-resorbable fibers.

Figure 18 is a schematic cross-section of a medical textile 1800 in which hyaluronan-based bioresorbable fibers 1810 and non-resorbable fibers 1816 are provided with another fiber 1820, which can be bioresorbable or non-resorbable. Any number of different kinds of hyaluronan-based bioresorbable fibers, and other fibers both hyaluronan-based and non-resorbable, are possible.

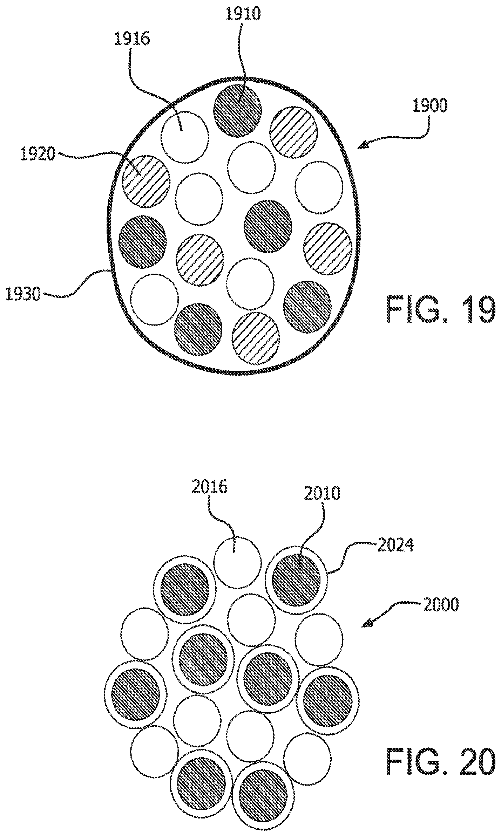

Figure 19 is a schematic cross-section of the medical textile 1900 which is comprised of hyaluronan-based bioresorbable fibers 1910, and non-resorbable fibers 1916, and possibly other fibers 1920 which can be hyaluronan-based bioresorbable fibers or non-resorbable fibers. The fiber bundle is surrounded by a coating of an active material 1930.

FIG 20 is a schematic cross-section of a medical textile 2000 in which hyaluronan-based bioresorbable fibers 2010 and non-resorbable fibers 2016 are provided. The hyaluronan-based bioresorbable fibers 210 are coated with an active material 2024. The active material 2024 could additionally or alternatively be provided on the non-resorbable fibers 2016, or on other bioresorbable fibers.

A method for repairing a portion of a mammalian body can include the step of providing a composite medical textile comprising a plurality of non-resorbable fibers and a plurality of bioresorbable hyaluronan-based fibers. The composite medical textile is manipulated to connect two tissue locations of a patient body, or possibly to connect a medical device to a portion of the patient body. In one embodiment, a composite suture according to the invention is provided and a suturing process is used to suture portions of the mammalian body. The medical textiles of the invention can be used in many different processes, such as connecting and repairing soft tissue and bony tissue, soft tissue augmentation, and stabilizing tissue of the joint. This method would allow for sufficient mechanical fixation and load carrying capability to allow the patient to begin nearly immediate post-operative rehabilitation activities to provide for an accelerated return to activity and minimize the potential for loss of motion or scarring following the surgical repair.

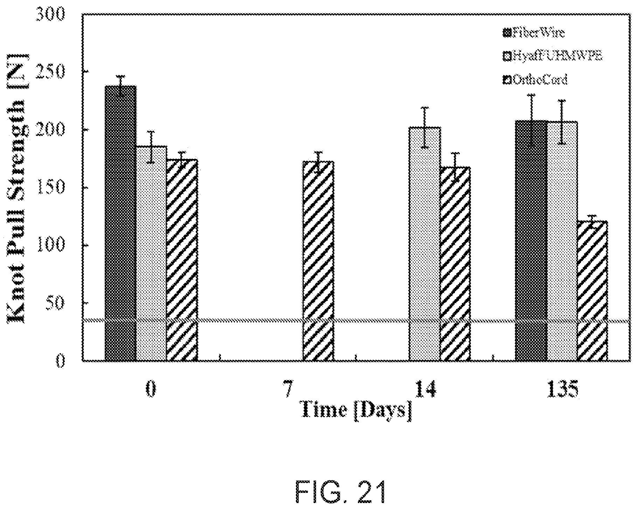

The tensile strength of composite Hyaff/UHMWPE suture and competitor products was evaluated by performing Knot-Pull testing according to USP <881 > Tensile Strength. Suture samples were each cut to ~20 cm in length, then placed in 50 mL 1X Phosphate Buffered Saline (PBS) at 37 C for: 1 hour (t=0), t days, 14 days, and 135 days to simulate physiological conditions. Peak knot pull strength was evaluated on a Mark X uniaxial tensile tester with a 5-cm gauge length tested at 100 mm/min crosshead speed. N=3 samples were tested for each group and the average ± standard deviation is shown in FIG. 21. The Orthocord (DePuy Synthes/Johnson&Johnson, Raynham MA) suture decreases in strength slightly out to 135 days, while the FiberWire (Arthrex, Naples FL) and Hyaff/UHMWPE samples exhibit no statistically significant decrease in Knot Pull strength between t=0 and t=135 days as the load is primarily carried by the UHMWPE portion of the composite suture. The horizontal line indicates the USP limit on average knot-pull for a Class I non-absorbable suture (34.5 N). The sutures of the invention demonstrate very good initial (to) knot pull strength and maintain knot pull strength at t=135 days.

The invention as shown in the drawings and described in detail herein disclose arrangements of elements of particular construction and configuration for illustrating preferred embodiments of structure and method of operation of the present invention. It is to be understood however, that elements of different construction and configuration and other arrangements thereof, other than those illustrated and described may be employed in accordance with the spirit of the invention, and such changes, alternations and modifications as would occur to those skilled in the art are considered to be within the scope of this invention as broadly defined in the appended claims. In addition, it is to be understood that the phraseology and terminology employed herein are for the purpose of description and should not be regarded as limiting. All references cited herein are hereby fully incorporated by reference.

### EMBODIMENTS

Although the present invention is defined in the attached claims, it should be understood that the present invention can also (alternatively) be defined in accordance with the following embodiments:
1. A composite medical textile, comprising a plurality of bioresorbable hyaluronan-based fibers and a plurality of non-resorbable fibers.
2. The composite medical textile of embodiment 1, wherein the bioresorbable hyaluronan-based fibers and the non-resorbable fibers are joined together by at least one selected from the group consisting of braided, knitted, adhered, intermeshed, weaved, interlocked, twisted, and heat set.
3. The composite medical textile of embodiment 1, wherein the hyaluronan-based fibers comprise at least one selected from the group consisting of hyaluronic acid, sodium hyaluronate, and esters of hyaluronic acid.
4. The composite medical textile of embodiment 3, wherein the esters of hyaluronic acid comprise benzyl esters of hyaluronic acid.
5. The composite medical textile of embodiment 1, wherein the medical textile comprises from 10 % to 98 % non-resorbable fibers, based on the weight of the non-resorbable fibers to the total weight of the non-resorbable suture fibers and bioresorbable hyaluronan-based polymer fibers.
6. The composite medical textile of embodiment 1, wherein the non-resorbable fibers comprise at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.
7. The composite medical textile of embodiment 1, further comprising bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxy butyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.
8. The composite medical textile of embodiment 1, wherein the hyaluronan-based bioresorbable polymer fibers are prepared by at least one selected from the group consisting of ring spinning, air-jet spinning, open-end spinning, mule spinning, wet spinning, dry spinning, electrospinning, pneumatospinning, pultrusion, and extrusion.
9. The composite medical textile of embodiment 8, wherein the extrusion comprises at least one selected from the group consisting of solvent exchange extrusion, precipitation extrusion, phase exchange extrusion, and phase change extrusion.
10. The composite medical textile of embodiment 1, wherein bioresorbable hyaluronan-based fiber comprises at least one selected from the group consisting of mono-and multifilaments.
11. The composite medical textile of embodiment 1, wherein the composite medical textile has a diameter of from 25 pm to about 5000 pm.
12. The composite medical textile of embodiment 1, further comprising at least one active agent.
13. The composite medical textile of embodiment 12, wherein the active agent is an antimicrobial agent.
14. The composite medical textile of embodiment 13, wherein the antimicrobial agent comprises at least one selected from the group consisting of Minocycline/Rifampicin, 5-Fluoro Uracil, Silver, Silver sulfadiazine, Penicillins, Tetracyclines, Cephalosporins, Cefazolins, Cefuroximes, Cefotoxins, Cefotaximines, Ceftazidimes, Cefalexins, Cefiximes, Carbapenems, Chlorhexidine, Triclosan, Levoflaxacin, Vancomycin, Imipenem, Cilastatin, Meropenem, Ciprofloxacin, Azithromycin, Clarithromycin, Sulfonamids, aminoglycosides, Quinolones, Lincomycins, Macrolides, Sulfonamides, and Glycopeptides.
15. The composite medical textile of embodiment 12, wherein the active agent is an analgesic.
16. The composite medical textile of embodiment 15, wherein the analgesic comprises at least one selected from the group consisting of Lidocaine, Bupivacaine, amylocaine, articaine, benzocaine, benzonatate, butacaine, butanilicaine, chloroprocaine, cinchocaine, cyclomethycaine, eucaine, ibuprofen, naproxen, paclitaxel, warfarin, heparin, tetracaine, dexamethasone, and ropivocaine.
17. The composite medical textile of embodiment 12, wherein the active agent comprises a vasoconstrictive agent.
18. The composite medical textile of embodiment 17, wherein the vasoconstrictive agent comprises at least one selected from the group consisting of epinephrine, alpha-adrenoreceptor antagonists, vasopressin analogues, norepinephrine, phenylephrine, dopamine, dobutamine, serotonin agonists, and triptans.
19. The composite medical textile of embodiment 12, wherein at least one selected from the group consisting of the non-resorbable fibers and the bioresorbable hyaluronan-based fibers are wetted with the active agent.
20. The composite medical textile of embodiment 12, wherein at least one selected from the group consisting of the non-resorbable fibers and the bioresorbable hyaluronan-based fibers are coated with the active agent.
21. The composite medical textile of embodiment 12, wherein at least one selected from the group consisting of the non-resorbable fibers and the bioresorbable hyaluronan-based fibers have the active agent embedded within the fiber.
22. The composite medical textile of embodiment 1, where the composite medical textile is circular in cross section.
23. The composite medical textile of embodiment 1, wherein the composite medical textile is a tape and has a cross section comprising at least one selected from the group consisting of rounded rectangular, marquise, oblong and oval.
24. A method of making a medical textile, comprising the steps of:
   providing a plurality of non-resorbable fibers;
   providing a plurality of bioresorbable hyaluronan-based fibers; and,
   joining the non-resorbable fibers and the bioresorbable hyaluronan-based fibers into a composite medical textile.
25. The method of embodiment 24, wherein the medical textile comprises from 20 % to 80 % bioresorbable hyaluronan-based fibers, based on the total number of non-resorbable fibers and bioresorbable hyaluronan-based fibers.
26. The method of embodiment 24, wherein the composite medical textile further comprises an active agent, the active agent comprising at least one selected from the group consisting of antimicrobial agents, analgesic agents, and vasoconstrictive agents.
27. The method of embodiment 24, wherein the active agent is coated onto at least one selected from the group consisting of the bioresorbable hyaluronan-based fibers and the non-resorbable fibers.
28. The method of embodiment 27, wherein the coating step comprises at least one selected from the group consisting of a dip, brush, spray, or curtain coating process.
29. The method of embodiment 24, wherein the active agent is impregnated into at least one selected from the group consisting of the bioresorbable hyaluronan-based fibers and the non-resorbable fibers by passing the extruded fiber through a solution containing the active agent.
30. The method of embodiment 24, wherein the active agent is impregnated into at least one selected from the group consisting of the bioresorbable hyaluronan-based fibers and the non-resorbable fibers by co-extrusion, wherein a fiber precursor and the active agent are combined into a homogeneous mixture and co-extruded into a fiber.
31. The method of embodiment 24, wherein at least one selected from the group consisting of the bioresorbable hyaluronan-based fibers and the non-resorbable fibers are wetted with the active agent.
32. A method for repairing a portion of a mammalian body, comprising the steps of:
   providing a composite medical textile, comprising a plurality of non-resorbable fibers and a plurality of bioresorbable hyaluronan-based fibers; and,
   connecting the textile between two tissue portions of the mammalian body.
33. The method of embodiment 32, wherein the tissue portions comprise at least one selected from the group consisting of bony tissue and soft tissue.
34. The method of embodiment 32, wherein the textile is a suture, and further comprising the step of manipulating the suture in a suturing process to suture the tissue portions of the mammalian body.
35. The method of embodiment 32, further comprising the step of seeding the medical textile with stem cells.
36. A medical device comprising a plurality of bioresorbable hyaluronan-based fibers and a plurality of non-resorbable fibers.
37. The medical device of embodiment 36, wherein the medical device is an orthopedic attachment system which comprises at least one flexible connector comprising bioresorbable hyaluronan-based fibers joined with a plurality of non-resorbable fibers, and at least one orthopedic attachment device.
38. The medical device of embodiment 36, wherein the medical device comprises at least one selected from the group consisting of tubes, membranes, non-woven fabrics, gauzes, sponges, and sutures.
39. The medical device of embodiment 36, wherein the medical device comprises a tissue scaffold.

## Claims

1. A medical device comprising a composite medical textile, said composite medical textile comprising a plurality of bioresorbable hyaluronan-based fibers and a plurality of non-resorbable fibers, wherein the bioresorbable hyaluronan-based fibers and the non-resorbable fibers are joined together by at least one selected from the group consisting of braided, knitted, intermeshed, weaved, interlocked, and twisted, and wherein the bioresorbable hyaluronan-based fibers and the non-resorbable fibers are optionally further joined together by at least one selected from the group consisting of adhered and heat set.

2. The medical device of claim 1, wherein the hyaluronan-based fibers comprise at least one selected from the group consisting of hyaluronic acid, sodium hyaluronate, and esters of hyaluronic acid.

3. The medical device of claim 2, wherein the esters of hyaluronic acid comprise benzyl esters of hyaluronic acid.

4. The medical device of claim 1, wherein the medical textile comprises from 10 % to 98 % non-resorbable fibers, based on the weight of the non-resorbable fibers to the total weight of the non-resorbable fibers and bioresorbable hyaluronan-based polymer fibers.

5. The medical device of claim 1, wherein the non-resorbable fibers comprise at least one selected from the group consisting of ultra-high molecular weight polyethylene (UHMWPE), polypropylene, polyethylene terephthalate (PET), polyethylene, polytetrafluoroethylene (Teflon), Dacron, steel, polybutester, polyamide, polyester, polyurethane, nylons, silk, and cotton.

6. The medical device of claim 1, further comprising bioresorbable fibers comprising at least one selected from the group consisting of collagen, polylactic acid (PLA), polyglycolic acid (PGA), polylactic-co-glycolic acid (PLGA), polycaprolactone (PCL), polydioxanone (PDO), polyhydroxy butyrate (PHB), polyhydroxyvalerate (PHV), Poly(3-hydroxybutyrate-co-3-hydroxyvalerate) (PHBV), alginate, chitosan, chitin, polylysine, fibrin, pectin, dextran, carrageenan, chondroitin sulfate, agar, gelatin, gellan gum, silk, and butyric acid.

7. The medical device of claim 1, wherein bioresorbable hyaluronan-based fiber comprises at least one selected from the group consisting of mono-and multifilaments.

8. The medical device of claim 1, wherein the composite medical textile has a diameter of from 25 µm to about 5000 µm.

9. The medical device of claim 1, further comprising at least one active agent, in particular an antimicrobial agent, an analgesic, or a vasoconstrictive agent.

10. The medical device of claim 1, where the composite medical textile is circular in cross section.

11. The medical device of claim 1, wherein the composite medical textile is a tape and has a cross section comprising at least one selected from the group consisting of rounded rectangular, marquise, oblong and oval.

12. The medical device of claim 1, wherein the composite medical textile is a composite suture.

13. The medical device of claim 12, wherein the medical device is a soft anchor assembly and wherein the composite suture passes through a flexible tubular anchor.

14. The medical device of claim 13, wherein the flexible tubular anchor tubular is comprised of a braided construction of hyaluronan-based bioresorbable fibers and non-resorbable fibers.

15. The medical device of claim 1, wherein the medical device is a soft anchor assembly comprising a suture and a flexible tubular anchor, wherein the suture passes through the flexible tubular anchor and wherein one of the tubular anchor and the suture is made completely from the non-resorbable fibers or from the hyaluronan-based bioresorbable fibers.
